(19) Europäisches Patentamt
European Patent Office
Office européen des brevets

(11) Publication number: **0 132 356**
**B1**

(12) **EUROPEAN PATENT SPECIFICATION**

(45) Date of publication of patent specification: **02.12.87**

(51) Int. Cl.$^4$: **C 07 D 205/08**

(21) Application number: **84304834.9**

(22) Date of filing: **16.07.84**

(54) **Process for preparing 2-azetidinones.**

(30) Priority: **18.07.83 US 514730**

(43) Date of publication of application:
**30.01.85 Bulletin 85/05**

(45) Publication of the grant of the patent:
**02.12.87 Bulletin 87/49**

(84) Designated Contracting States:
**AT BE CH DE FR GB IT LI LU NL SE**

(56) References cited:
**US-A-3 991 069**

**THE JOURNAL OF ORGANIC CHEMISTRY, vol. 47, no. 1, 1st January 1982, pages 179-180, American Chemical Society, USA; C.M. CIMARUSTI et al.: "Monobactams. The conversion of 6-APA to (S)-3-amino-2-oxoazetidine-1-sulfonic acid and its 3(RS)-methoxy derivative"**

(73) Proprietor: **E.R. Squibb & Sons, Inc.**
**Lawrenceville-Princeton Road**
**Princeton, N.J. 08540 (US)**

(72) Inventor: **Koster, William H.**
**Box 409, R.D. No. 3 Welisewitz Rd.**
**Ringoes New Jersey (US)**

(74) Representative: **Thomas, Roger Tamlyn et al**
**D. Young & Co. 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 132 356 B1**

**Description**

A compound having the formula

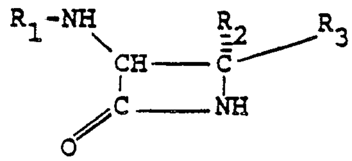

I

can be prepared by heating together an anion having the formula

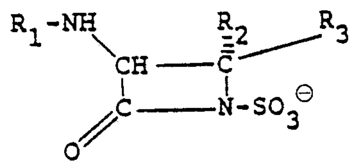

II

and a cation having the formula

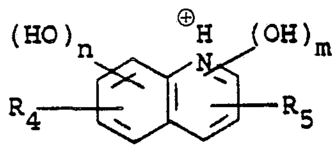

III

or

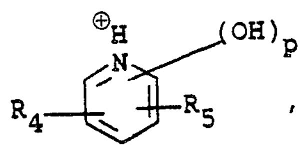

IV

provided, however, that if in formula III n and m are each O or in formula IV p is 0, a nucleophile having the formula

$$R_6\text{—OH} \qquad \text{V}$$

must also be present. The reaction is effected in the presence of an organic solvent (which may be the nucleophile $R_6$—OH).

As used in the above formulas, and through-out the specification, the symbols are as defined below:

$R_1$ is an acyl group derived from a carboxylic acid;

$R_2$ and $R_3$ are the same or different and each is hydrogen, alkyl, alkenyl, alkynyl, cycloalkyl, phenyl, substituted phenyl or a 4, 5, 6 or 7-membered heterocycle (referred to hereinafter as $R_x$) or one of $R_2$ and $R_3$ is hydrogen and the other is azido, halomethyl, dihalomethyl, trihalomethyl, alkoxycarbonyl, 2-phenylethenyl, 2-phenylethynyl, carboxyl, —$CH_2X_1$ [wherein $X_1$ is azido, amino (—$NH_2$), hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano,

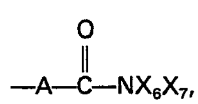

—S—$X_2$, or O—$X_2$ (wherein A, $X_2$, $X_6$ and $X_7$ are as hereinafter defined)], —S—$X_2$ or O—$X_2$ [wherein $X_2$ is alkyl, substituted alkyl, phenyl, substituted phenyl, phenylalkyl, (substituted phenyl)alkyl, alkanoyl, phenylalkanoyl, (substituted phenyl)alkanoyl, phenylcarbonyl, (substituted phenyl)carbonyl, or heteroarylcarbonyl],

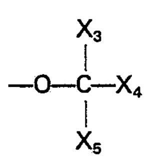

or

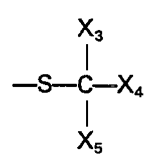

[wherein one of $X_3$ and $X_4$ is hydrogen and the other is hydrogen or alkyl, or $X_3$ and $X_4$ when taken together with the carbon atom to which they are attached form a cycloalkyl group; and $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl

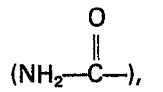

(substituted amino)-carbonyl, or cyano (—C≡N)], or

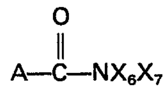

(wherein A is —CH=CH—, $-(CH_2)_n-$, $-CH_2-O-$, $-CH_2-NH-$, or $-CH_2-S-CH_2-$, n is 0, 1 or 2, and $X_6$ and $X_7$ are the same or different and each is hydrogen, alkyl, phenyl or substituted phenyl, or $X_6$ is hydrogen and $X_7$ is amino, substituted amino, alkanoylamino or alkoxy, or $X_6$ and $X_7$ when taken together with the nitrogen atom to which they are attached form 4, 5, 6 or 7-membered heterocycle);

$R_4$ and $R_5$ are the same or different and each is hydrogen, alkyl, substituted alkyl, phenyl, substituted phenyl, halogen, alkoxy, phenyloxy, (phenylcarbonyl)oxy, alkanoyloxy, alkanoylamino, or (phenylcarbonyl)amino;

$R_6$ is hydrogen, alkyl, or phenyl;

n is 0 or 1, m is 0 or 1 and the sum of n + m is 0 or 1; and

p is 0 or 1.

Listed below are definitions of various terms used to describe the β-lactams of this invention. These definitions apply to the terms as they are used throughout the specification (unless they are otherwise limited in specific instances) either individually or as part of a larger group.

The terms "alkyl" and "alkoxy" refer to both straight and branched chain groups having 1 to 10 carbon atoms.

The terms "cycloalkyl" and "cycloalkenyl" refer to cycloalkyl and cycloalkenyl groups having 3, 4, 5, 6 or 7 carbon atoms.

The term "substituted alkyl" refers to alkyl groups substituted with one, or more, azido, amino ($-NH_2$), halogen, hydroxy, carboxyl, cyano, alkoxycarbonyl, aminocarbonyl, alkanoyloxy, alkoxy, phenyloxy, (substituted phenyl)oxy, $R_x$-oxy, mercapto, alkylthio, phenylthio, (substituted phenyl)thio, alkylsulfinyl, or alkylsulfonyl groups.

The terms "alkanoyl", "alkenyl", and "alkynyl" refer to both straight and branched chain groups having 2 to 10 carbon atoms.

The terms "halogen" and "halo" refer to fluorine, chlorine, bromine and iodine.

The term "protected carboxyl" refers to a carboxyl group which has been esterified with a conventional acid protecting group. These groups are well known in the art; see, for example, United States patent 4,144,333, issued March 13, 1979. The preferred protected carboxyl groups are benzyl, benzhydryl, t-butyl, and p-nitrobenzyl esters.

The term "substituted phenyl" refers to a phenyl group substituted with 1, 2 or 3 amino ($-NH_2$), halogen hydroxyl, trifluoromethyl, alkyl (of 1 to 4 carbon atoms), alkoxy (of 1 to 4 carbon atoms), or carboxyl groups.

The term "substituted amino" refers to a group having the formula $-NY_1Y_2$ wherein $Y_1$ is hydrogen, alkyl, phenyl, substituted phenyl, phenylalkyl or (substituted phenyl)alkyl, and $Y_2$ is alkyl, phenyl, substituted phenyl, phenylalkyl, (substituted phenyl)alkyl, hydroxy, cyano, alkoxy, phenylalkoxy, or amino ($-NH_2$).

The expression "a 4,5,6 or 7-membered heterocycle" (referred to as "$R_x$") refers to pyridinyl, furanyl, pyrrolyl, thienyl, 1,2,3-triazolyl, 1,2,4-triazolyl, imidazolyl, thiazolyl, thiadiazolyl, pyrimidinyl, oxazolyl, triazinyl, tetrazolyl, azetinyl, oxetanyl, thietanyl, piperidinyl, piperazinyl, imidazolidinyl, oxazolidinyl, pyrrolidinyl, tetrahydropyrimidinyl, dihydrothiazolyl and hexahydroazepinyl or one of the above groups substituted with one or more oxo (=O), halogen, hydroxy, nitro, amino, cyano, trifluoromethyl, alkyl of 1 to 4 carbons, alkoxy of 1 to 4 carbons, alkylsulfonyl, phenyl, substituted phenyl, 2-furfurylideneamino

( 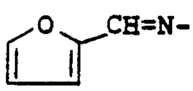 ),

benzylideneimino and substituted alkyl groups (wherein the alkyl group has 1 to 4 carbons).

Exemplary of the substituted 4,5,6 or 7-membered heterocycles are 1-alkyl-3-azetinyl, 2-oxo-1-imidazolidinyl, 3-alkylsulfonyl-2-oxo-1-imidazolidinyl, 3-benzylimino-2-oxo-1-imidazolinyl, 3-alkyl-2-oxo-1-imidazolidinyl, 3-phenyl (or substituted phenyl)-2-oxo-1-imidazolidinyl, 3-benzyl-2-oxo-1-imidazolidinyl, 3-(2-aminoethyl)-2-oxo-1-imidazolidinyl, 3-amino-2-oxo-1-imidazolidinyl, 3-[(alkoxycarbonyl)amino]-2-oxo-1-imidazolidinyl, 3-[2-[(alkoxycarbonyl)amino]ethyl]-2-oxo-1-imidazolidinyl, 2-oxo-1-pyrrolidinyl, 2-oxo-3-oxazolidinyl, 4-hydroxy-6-methyl-2-pyrimidinyl, 2-oxo-1-hexahydroazepinyl, 2-oxo-3-pyrrolidinyl, 2-oxo-3-tetrahydrofuranyl, 2,3-dioxo-1-piperazinyl, 2,5-dioxo-1-piperazinyl, 4-alkyl-2,3-dioxo-1-piperazinyl, and 4-phenyl-2,3-dioxo-1-piperazinyl.

The term "acyl" refers to all organic radicals derived from an organic acid (*i.e.*, a carboxylic acid) by removal of the hydroxyl group. Certain acyl groups are, of course, preferred but this preference should not be viewed as a limitation of the scope of this invention. Exemplary acyl groups are those acyl groups which have been used in the past to acylate β-lactam antibiotics including 6-aminopenicillanic acid and derivatives and 7-aminocephalosporanic acid and derivatives; see, for example, *Cephalosporins and Penicillins*, edited by Flynn, Academic Press (1972), German Offenlegungsschrift 2,716,677, published October 10, 1978, Belgian patent 867,994, published December 11, 1978, United States patent 4,152,432, issued May 1, 1979, United States patent 3,971,778, issued July 27, 1976, United States patent 4,172,199, issued October 23, 1979, and British patent 1,348,894, published March 27, 1974. The portions of these references describing various acyl groups are incorporated herein by reference. The following list of acyl groups is presented to further exemplify the term "acyl"; it should not be regarded as limiting that term. Exemplary acyl groups are:

(a) Aliphatic groups having the formula

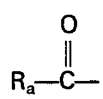

wherein $R_a$, is alkyl; cycloalkyl; alkoxy; alkenyl; cycloalkenyl; cyclohexadienyl; or alkyl or alkenyl substituted with one or more halogen, cyano, nitro, amino, mercapto, alkylthio, or cyanomethylthio groups.

(b) Carbocyclic aromatic groups having the formula

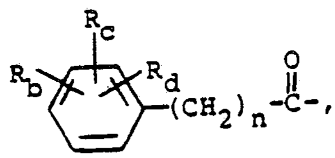 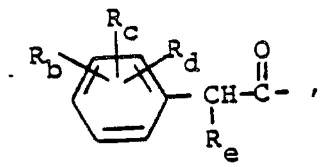

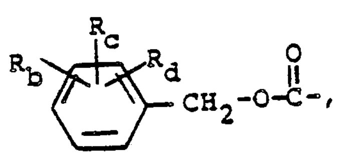 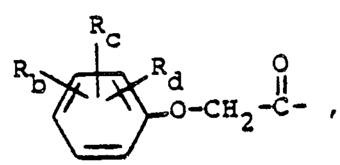

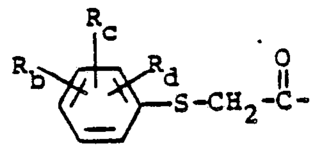 or 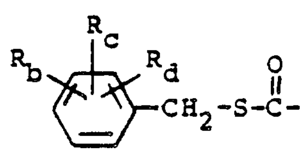

wherein n is 0, 1, 2 or 3; $R_b$, $R_c$, and $R_d$ each is independently hydrogen, halogen, hydroxyl, nitro, amino, cyano, trifluoromethyl, alkyl of 1 to 4 carbon atoms, alkoxy of 1 to 4 carbon atoms or aminomethyl; and $R_e$ is amino, hydroxyl, a carboxyl salt, protected carboxyl, formyloxy, a sulfo salt, a sulfoamino salt, azido, halogen, hydrazino, alkylhydrazino, phenylhydrazino, or [(alkylthio)thioxomethyl]thio.

Preferred carbocyclic aromatic acyl groups include those having the formula

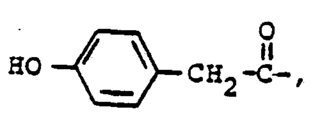 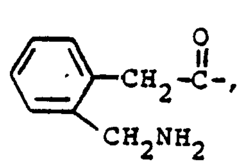 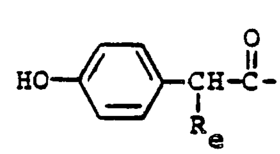

($R_e$ is preferably a carboxyl salt or sulfo salt) and

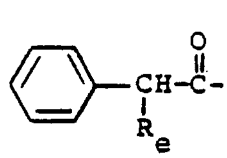

($R_e$ is preferably a carboxyl salt or sulfo salt).

(c) Heteroaromatic groups having the formula

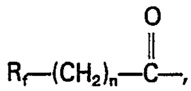, 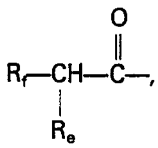, 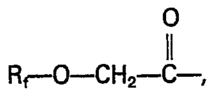, 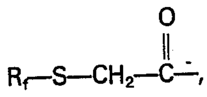, 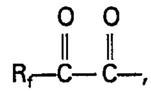

wherein n is 0, 1, 2 or 3; $R_e$ is as defined above; and $R_f$ is a substituted or unsubstituted 5-, 6- or 7-membered heterocyclic ring containing 1,2,3 or 4 (preferably 1 or 2) nitrogen, oxygen and sulfur atoms. Exemplary heterocyclic rings are thienyl, furyl, pyrrolyl, pyridinyl, pyrazolyl, pyrazinyl, thiazolyl, pyrimidinyl, thiadiazolyl and tetrazolyl. Exemplary substituents are halogen, hydroxyl, nitro, amino, protected amino, cyano, trifluoromethyl, alkyl of 1 to 4 carbon atoms, alkoxy of 1 to 4 carbon atoms, or

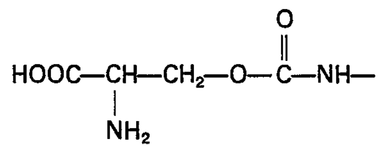.

Preferred heteroaromatic acyl groups include those groups of the above formulas wherein $R_f$ is 2-amino-4-thiazolyl, 2-amino-5-halo-4-thiazolyl, 4-aminopyrimidin-2-yl, 5-amino-1,2,4-thiadiazol-3-yl, 2-thienyl, 2-furanyl, or 6-aminopyridin-2-yl.

(d) [[(4-Substituted-2,3-dioxo-1-piperazinyl)carbonyl]amino]arylacetyl groups having the formula

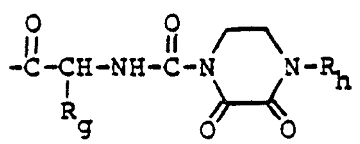

wherein $R_g$ is an aromatic group (including carbocyclic aromatics such as those of the formula

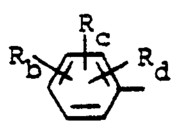

and heteroaromatics as included within the definition of $R_f$); and $R_h$ is alkyl, substituted alkyl (wherein the alkyl group is substituted with one or more halogen, cyano, nitro, amino or mercapto groups), arylmethyleneamino (*i.e.*, $-N=CH-R_g$ wherein $R_g$ is as defined above), arylcarbonylamino (*i.e.*,

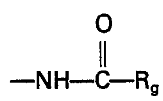

wherein $R_g$ is as defined above) or alkylcarbonylamino.

Preferred [[(4-substituted-2,3-dioxo-1-piperazinyl)carbonyl]amino]arylacetyl groups include those wherein $R_h$ is ethyl, phenylmethyleneamino or 2-furylmethyleneamino.

(e) (Substituted oxyimino)arylacetyl groups having the formula

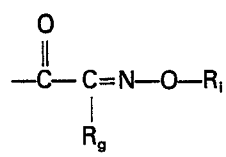

wherein $R_g$ is as defined above and $R_i$ is hydrogen, alkyl, cycloalkyl, alkylaminocarbonyl, arylaminocarbonyl (*i.e.*,

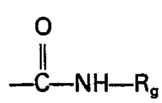

wherein $R_g$ is as defined above) or substituted alkyl (wherein the alkyl group is substituted with one or more

halogen, cyano, nitro, amino, mercapto, alkylthio, aromatic group (as defined by $R_g$), carboxyl (including salts thereof), amido, alkoxycarbonyl, phenylmethoxycarbonyl, diphenylmethoxycarbonyl, hydroxyalkoxyphosphinyl, dihydroxyphosphinyl, hydroxy(phenylmethoxy)phosphinyl, dialkoxyphosphinyl or tetrazolyl substituents).

Preferred (substituted oxyimino)arylacetyl groups include those wherein $R_g$ is 2-amino-4-thiazolyl. Also preferred are those groups wherein $R_l$ is methyl, ethyl, carboxymethyl, 1-carboxy-1-methylethyl, 2,2,2-trifluoroethyl or 1-carboxycyclopropyl.

(f) (Acylamino)arylacetyl groups having the formula

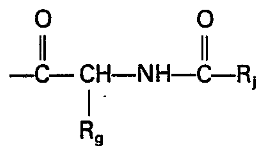

wherein $R_g$ is as defined above and $R_j$ is

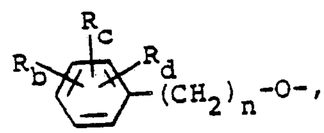

amino, alkylamino, (cyanoalkyl)amino, amido, alkylamido, (cyanoalkyl)amido,

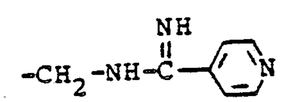
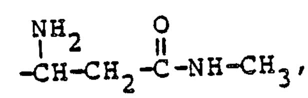,

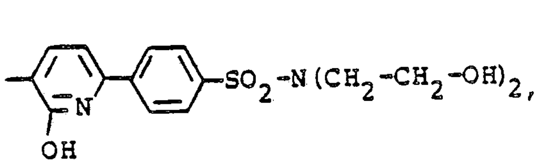,
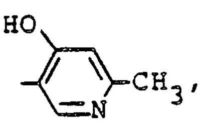,

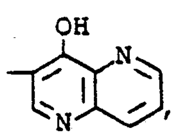,
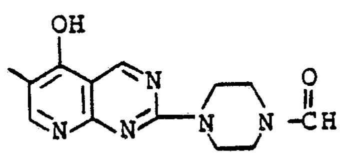, or
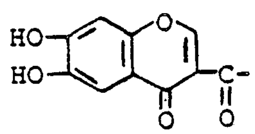.

Preferred (acylamino)arylacetyl groups of the above formula include those groups wherein $R_j$ is amino or amido. Also preferred are those groups wherein $R_g$ is phenyl or 2-thienyl.

(g) [[[3-Substituted-2-oxo-1-imidazolidinyl]carbonyl]amino]arylacetyl groups having the formula

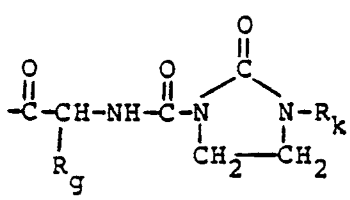

wherein $R_g$ is as defined above and $R_k$ is hydrogen, alkylsulfonyl, arylmethyleneamino (*i.e.*, —N=CH—$R_g$ wherein $R_g$ is as defined above),

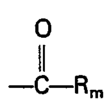

(wherein $R_m$ is hydrogen, alkyl or halogen substituted alkyl), aromatic group (as defined by $R_g$ above), alkyl or substituted alkyl (wherein the alkyl group is substituted with one or more halogen, cyano, nitro, amino or mercapto groups).

Preferred [[3-substituted-2-oxo-1-imidazolidinyl]carbonyl]amino]arylacetyl groups of the above formula include those wherein $R_g$ is phenyl or 2-thienyl. Also preferred are those groups wherein $R_k$ is hydrogen, methylsulfonyl, phenylmethyleneamino or 2-furylmethyleneamino.

The terms "salts" and "salts", when used to describe a β-lactam, refer to basic salts formed with inorganic and organic bases. Such salts include ammonium salts, alkali metal salts like sodium and potassium salts, alkaline earth metal salts like the calcium and magnesium salts, salts with organic bases, e.g., dicyclohexylamine salt, benzathine, N-methyl-D-glucamine, hydrabamine salts, salts with amino acids like arginine, lysine and the like. The nontoxic, pharmaceutically acceptable salts are preferred, although other salts are also useful, because this invention deals with the preparation of chemical intermediates, not final products to be used as medicines.

Salts of an azetidinone-1-sulfonic acid are formed by reacting the free acid form of the sulfonate with one or more equivalents of an appropriate base providing the desired cation in water or in a solvent mixture containing water. The salt is isolated by removal of solvent *in vacuo* or, in the case of water, by lyophilization. The free acid of the sulfonate is formed by treating an azetidinone-1-sulfonic acid salt with an insoluble sulfonic acid such as cation exchange resin in the hydrogen form (e.g. a polystyrene sulfonic acid resin like *Dowex* (Trade Mark) 50).

Alternatively, salts may be formed by cation interchange. A salt of a β-lactam compound soluble in an organic solvent is combined with a salt containing the desired cation, also soluble in the same solvent system. The solvent system is chosen so that the formed salt is much less soluble than either of the added salts and thus precipitates from the medium and is collected.

The terms "salts" and "salts", when used to describe a quinoline compound or pyridine compound refer to acid-addition salts formed with inorganic and organic acids. Illustrative salts are the hydrohalides (especially the hydrochloride and hydrobromide), sulfate, nitrate, phosphate, tartrate, maleate, citrate, salicylate, methanesulfonate, benzenesulfonate, toluenesulfonate and the like.

It has been found that a cation of formula III or IV, which contains a hydroxy substituent, will associate with the sulfonate anion of formula II and then serve as a nucleophile and protic source for removal of the sulfonate group. Alternatively, an external nucleophile (*i.e.*, a compound of formula V) can be used in conjunction with a cation of formula III or IV which does not contain a hydroxy substituent. This new methodology for the preparation of compounds of formula I affords a general synthesis for the preparation of monocyclic β-lactam antibiotics.

In one embodiment of this invention, both the anion of formula II and cation of formula III or IV are in the form of a salt having the formula

VI 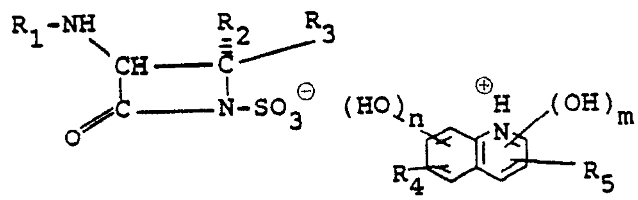

or

VII 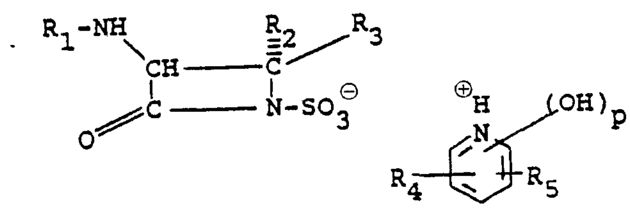

A salt of formula VI or VII can be heated in an organic solvent (e.g., ethanol, acetone, acetonitrile or others in which it has some solubility) to yield the desired 2-azetidinone of formula I. In the case of a salt wherein the cationic portion does not contain a hydroxy group, the heating must be done in the presence of a nucleophile of formula V.

In another embodiment of this invention, the anion of formula II is in the form of a salt of a compound having the formula

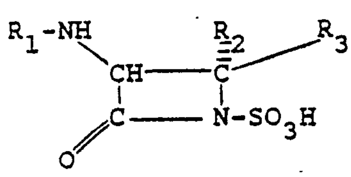 VIII

and the cation of formula III or IV is in the form of a salt of a compound having the formula

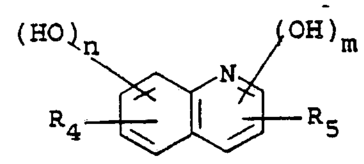

IX

or

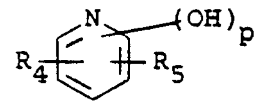

X

The salt of a compound of formula VIII can be dissolved in an organic solvent, combined with a salt of formula IX or X and heated to yield the desired 2-azetidinone of formula I. In those reactions wherein the salts of formulas IX and X do not contain a hydroxy group, the heating must be done in the presence of a nucleophile of formula V.

A salt of formula VI or VII can be prepared using known methodology from β-lactam compounds of formula VIII and salts thereof. The β-lactam compounds of formula VIII exist in their most stable form as salts (including inner salts). In dilute aqueous solution, they also exist as acids. The acid form, in an aqueous solution, can be obtained by passage of a corresponding salt through an ion-exchange resin (acid form).

If the acid form of a compound of formula VIII is used as a precursor to a compound of formula VI or VII, a compound of formula IX or X is first added to a solution of the acid in water containing a miscible organic solvent (*e.g.*, acetone or acetonitrile), which upon removal of the solvent mixture yields the corresponding salt.

A salt of formula VI or VII can also be prepared from salts of compounds of formula VIII using the cation interchange methodology described above under the description of the terms "salt" and "salts".

The following examples are specific embodiments of this invention.

## Example 1
## (3S-trans)-3-[[(Phenylmethoxy)carbonyl]amino]-4-methyl-2-azetidinone

### Method I

A) (3S-trans)-2-Oxo-3-[[(phenylmethoxy)carbonyl]amino]-4-methyl-1-azetidinesulfonic acid, 8-hydroxyquinolinium salt

(3S-trans)-2-Oxo-3-[[(phenylmethoxy)carbonyl]amino]-4-methyl-1-azetidinesulfonic acid, tetrabutylammonium salt (500 mg, 0.9 mmol) was dissolved in a mixture of water and acetonitrile. The solution was passed through an AGMP—50 ion-exchange resin column ($H^{\oplus}$ form, 7 ml, 1.7 meq/ml) eluting with water. The eluate was run into a flask containing 8-hydroxyquinoline (131 mg, 0.9 mmol). Acetonitrile was removed from the resulting solution under reduced pressure at <35°C and the remaining aqueous solution was lyophilized yielding a solid. Trituration with ether and drying *in vacuo* afforded the desired product as a yellow powder (398 mg), melting point 61—68°C.

| | | | | |
|---|---|---|---|---|
| Analysis Calc'd. for $C_{21}H_{21}N_3O_7S{\cdot}0.7H_2O$ (472.17): | C, 53.42; | H, 4.78; | N, 8.90; | S, 6.79 |
| Found: | C, 53.42; | H, 4.48; | N, 8.98; | S, 6.68 |

B) (3S-trans)-3-[[(Phenylmethoxy)carbonyl]amino]-4-methyl-2-azetidinone

A solution of (3S-trans)-2-oxo-3-[[(phenylmethoxy)carbonyl]amino]-4-methyl-1-azetidinesulfonic acid, 8-hydroxyquinolinium salt (150 mg, 0.326 mmol) in acetonitrile (10 ml) was brought repidly to reflux by lowering the reaction vessel into a preheated oil bath. The mixture was refluxed for a total of 9 minutes during which time a precipitate formed. After cooling to room temperature, the mixture was filtered and solvent was removed from the filtrate *in vacuo*. The resulting residue was partially dissolved in a small amount of acetonitrile and the addition of ethyl acetate caused further precipitation. After decanting the supernatant, the procedure was repeated on the solid. The remaining solid was triturated with ethyl acetate and decanted. The supernatants were filtered through *Celite*, (Trade Mark) combined, and solvent was removed under reduced pressure yielding an oil (84 mg). The crude product was purified by colum chromatography on silica gel (2 g, Mallinckrodt *SilicAR* (Trade Mark) CC—4). Elution with 5% acetonitrile: 95% ethyl acetate afforded the desired product (56 mg).

After crystallization from ether: m.p. 100—101°C.

| | | | |
|---|---|---|---|
| Analysis Calc'd. for $C_{12}H_{14}N_2O_3$: | C, 61.52; | H, 6.02; | N, 11.96 |
| Found: | C, 61.52; | H, 5.88; | N, 12.00 |

IR($CH_2Cl_2$): 1770 $cm^{-1}$ (β-lactam C=O)

NMR ($CD_3CH$): δ 1.35 (d, J=3.0, 6.0 Hz, 3H) 3.7 (dq, J=2.5, 6.0 Hz, 1H), 4.2 (dd, J=2.5, 8.0 Hz, 1H), 5.15 (5,2H), 7.4 (5, 5H).

### Method II

Following the procedure of Method I, part A, but utilizing 4-hydroxyquinoline in place of 8-hydroxyquinoline, yielded (3S-*trans*)-3-[[(phenylmethoxy)carbonyl]amino]-4-methyl-1-azetidinesulfonic acid, 4-hydroxyquinolinium salt as a colorless solid (after lyophilization), melting point 58—66°C.

Analysis Calc'd. for $C_{21}H_{21}N_3O_7S$ (MW 459.47): C, 54.89; H, 4.69; N, 9.14; S, 6.98
Found: C, 54.81; H, 4.81; N, 9.24; S, 6.34

This salt (46 mg) was heated at reflux in acetonitrile (2 ml) for 4 hours. Formation of the title compound was verified by thin layer chromatography (Merck silica gel 60F, 3:1:1 butanol:acetic acid:water).

Method III

Following the procedure of Method I, part A, but utilizing 5-hydroxyquinoline in place of 8-hydroxyquinoline, yielded (3S-*trans*)-3-[[(phenylmethoxy)carbonyl]amino]-4-methyl-1-azetidinesulfonic acid, 5-hydroxyquinolinium salt, melting point 55—64°C.

Analysis Calc'd. for $C_{21}H_{21}N_3O_7S \cdot 0.55H_2O$ (469.44): C, 53.73; H, 4.75; N, 8.95
Found: C, 53.73; H, 4.59; N, 9.18

This salt (115 mg) was dissolved in dry acetonitrile (5 ml) and the resulting solution was heated at reflux for 0.5 hours. After cooling, solvent was removed *in vacuo* and the residue was dissolved in an ethyl acetate (6 ml)-water (3 ml) mixture. After addition of magnesium sulfate (68 mg) and sodium bicarbonate (63 mg), the mixture was shaken. The organic layer was separated, washed with water (3 ml), and dried (magnesium sulfate). Solvent was removed *in vacuo*, the residue was dissolved in acetonitrile, and the insolubles were removed by filtration. Solvent was removed under reduced pressure and the residue was chromatographed on silica gel (Mallinckrodt SilicAr CC—7). Elution with ethyl acetate yielded (3S-*trans*)-2-oxo-3-[[(phenylmethoxy)carbonyl]amino]-4-methyl-2-azetidinone (27 mg).

Method IV

A) (3S-*trans*))-2-Oxo-3-[[(phenylmethoxy)carbonyl)amino]-4-methyl-1-azatidinesulfonic acid, pyridinium salt

Following the procedure of Method I, part A, but utilizing pyridine in place of 8-hydroxyquinoline, yielded (3S-*trans*)-3-[[(phenylmethoxy)carbonyl]amino]-4-methyl-1-azetidinesulfonic acid, pyridinium salt as a colorless solid (after lyophilization), melting point 56—64°C.

Analysis Calc'd. for $C_{16}H_{17}N_3O_6$ (MW 379.99): C, 50.65; H, 4.52; N, 11.08; S, 8.45
Found: C, 50.33; H, 4.55; N, 10.80; S, 8.09

B) (3S-*trans*)-3-[[Phenylmethoxy)carbonyl]amino]-4-methyl-2-azetidinone

(3S-*trans*)-2-Oxo-3-[[(phenylmethoxy)carbonyl]amino]-4-methyl-1-azetidinesulfonic acid, pyridinium salt (190 mg, 0.5 mmol) was dissolved in dry acetonitrile (10 ml), phenol (47 mg, 0.5 mmol) was added, and the mixture was refluxed for 4 hours. Solvent was removed *in vacuo*, the residue was dissolved in an ethyl acetate-water mixture and shaken after addition of magnesium sulfate (136 mg) and sodium bicarbonate (126 mg). The ethyl acetate layer was separated, washed with water, and dried (magnesium sulfate). Solvent was removed *in vacuo*, and the residue was purified by column chromatography on silica gel (Mallinckrodt SilicAr CC—7). Elution with ethyl acetate yielded (3S-*trans*)-2-oxo-3-[[(phenylmethoxy)-carbonyl]amino]-4-methyl-2-azetidinone (24 mg).

Method V

(3S-*trans*)-2-Oxo-3-[[(phenylmethoxy)carbonyl)amino]-4-methyl-1-azetidinesulfonic acid, pyridinium salt (393 mg, 0.1 mmol; see Method IVA) was dissolved in absolute ethanol (20 ml) and refluxed for 4 hours. The reaction mixture was cooled to room temperature and ethanol was removed *in vacuo*. The residue was purified by column chromatography on silica gel (Mallinckrodt SilicAr CC—7). Elution with ethyl acetate yielded (3S-*trans*)-2-oxo-3-[[(phenylmethoxy)carbonyl]amino]-4-methyl-2-azetidinone as an oil (152 mg).

Method VI

(3S-trans)-2-Oxo-3-[[(phenylmethoxy)carbonyl]amino]-4-methyl-1-azetidinesulfonic acid, 8-hydroxyquinolinium salt (230 mg, 0.5 mmol; see Method IA) was dissolved in absolute ethanol (10 ml) and refluxed for 4 hours. Ethanol was removed *in vacuo* yielding a residue which was triturated with ethyl acetate (5 × 10 ml). From the combined extract, solvent was removed *in vacuo* affording a residue which was chromatographed on silica gel (Mallinckrodt SilicAr CC—7). Elution with ethyl acetate yielded (3S-*trans*)-3-[[(phenylmethoxy)carbonyl]amino]-4-methyl-2-azetidinone (98 mg).

Method VII

(3S-*trans*)-2-Oxo-3-[[(phenylmethoxy)carbonyl]amino]-4-methyl-1-azetidinesulfonic acid, 5-hydroxyquinolinium salt (230 mg, 0.5 mmol; see Method III) was treated as described in Method VI to yield the desired (3S-*trans*)-3-[[(phenylmethoxy)carbonyl]amino]-4-methyl-2-azetidinone (87 mg).

Example 2

(3S)-3-[[(Phenylmethoxy)carbonyl]amino]-2-azetidinone

Method I

A) (3S)-2-Oxo-3-[[(phenylmethoxy)carbonyl]amino]-1-azetidinesulfonic acid, 8-hydroxyquinolinium salt

From (3S)-2-oxo-3-[[(phenylmethoxy)carbonyl]amino]-1-azetidinesulfonic acid, tetrabutylammonium salt (600 mg, 1.13 mmol) the desired product was obtained as a yellow powder (467 mg) following the procedure described in Example 1A, method I, part A.

B) (3S)-3-[[(Phenylmethoxy)carbonyl]amino]-2-azetidinone

(3S)-2-Oxo-3-[[(phenylmethoxy)carbonyl]amino]-1-azetidinesulfonic acid, 8-hydroxyquinolinium salt (150 mg, 0.337 mmol) was added to acetonitrile (10 ml) and rapidly heated to reflux in a preheated oil bath. After refluxing for a total of 18 minutes, the mixture was cooled to room temperature, filtered through Celite, and solvent was removed *in vacuo*. The residue was extracted with an acetonitrile-ethyl acetate mixture and the extract chromatographed on a silica gel column (Mallinckrodt SilicAr CC—7). The desired product (31 mg) was eluted with a 5% acetonitrile:95% ethyl acetate mixture, m.p. 163—164°C.

| | | | |
|---|---|---|---|
| Analysis calc'd. for $C_{11}H_{12}N_2O_3$: | C, 59.97; | H, 5.50; | N, 12.72 |
| Found: | C, 60.47; | H, 5.43; | N, 20.7 |

$[\alpha]_D$: −18.2 (C=1.00, MeOH)

Method II

A) 8-Hydroxyquinolinium, p-toluenesulfonate

The toluenesulfonic acid salt of 8-hydroxyquinoline was formed by dissolving toluenesulfonic acid monohydrate (1.9 g, 10 mmol) in acetonitrile and adding 8-hydroxyquinoline (1.45 g, 10 mmol). Removal of solvent *in vacuo* and addition of ether to the residual oil results in crystallization. Trituration several times with ether followed by drying *in vacuo* yields 8-hydroxyquinolinium, p-toluenesulfonate as a powder (3.11 g).

B) (3S)-3-[[(Phenylmethoxy)carbonyl]amino]-2-azetidinone

To a solution of (3S)-2-oxo-3-[[(phenylmethoxy)carbonyl]amino]-1-azetidinesulfonic acid, tetrabutylammonium salt in acetonitrile was added 8-hydroxyquinolinium p-toluenesulfonate. The mixture was refluxed, yielding the title compound as verified by thin layer chromatography (Merck silica gel 60F, ethyl acetate).

Method III

A) (3S)-2-Oxo-3-[[(Phenylmethoxy)carbonyl]amino]-1-azetidinesulfonic acid, 8-hydroxyquinolinium salt

To a stirred solution of 3.58 g of (3S)-2-oxo-3-[[(phenylmethoxy)carbonyl]amino]-1-azetidinesulfonic acid, tetrabutylammonium salt and 960 mg of 8-hydroxyquinoline in 10 ml of dichloroethane was added 1.53 g of camphorsulfonic acid. An additional 5 ml of dichloroethane was added and the mixture was stirred to effect solution and a seed crystal of the desired product was added to initiate precipitation. After ca. 5 minutes, 15 ml of ethyl acetate was added slowly. After an additional 30 minutes, the solid was isolated by filtration, washed with 1:1 dichloromethane/ethyl acetate and dried *in vacuo* to afford 1.98 g of the title compound as a fine, light yellow powder.

B) (3S)-3-[[(Phenylmethoxy)carbonyl]amino]-2-azetidine

A stirred suspension of 1.00 g of (3S)-2-oxo-3-[[(phenylmethoxy)carbonyl]amino]-1-azetidinesulfonic acid, 8-hydroxyquinolinium salt in 75 ml of anhydrous ethanol under nitrogen was placed in a 95°C oil bath. The mixture was refluxed for 20 minutes, then cooled to 20°C with an ice water bath. The solvent was removed on the rotary evaporator (bath temperature ≤30°C). The residue was taken up in 10 ml of water and 80 ml of ethyl acetate. About 600 mg of $MgSO_4$ was added followed by 570 mg of sodium bicarbonate. The mixture was stirred to effect solution. The organic layer was separated, washed with 5 ml of water, and dried over $MgSO_4$. The solvent was evaporated to afford a white solid, which was transferred to a 25 ml flask with acetone. The acetone was removed *in vacuo* and the solid residue was suspended in ca. 5 ml of ethyl acetate. Ether (ca. 6 ml) was added and the mixture was stored for 60 minutes at 0°C. The solid was isolated by filtration, washed with ether, and dried *in vacuo* to afford 392 mg of the title compound as a white solid.

**Claims**

1. A process for preparing a compound having the formula

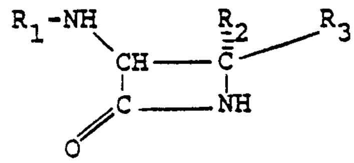

which comprises heating an anion having the formula

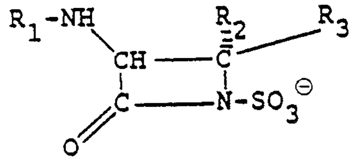

with a cation having the formula

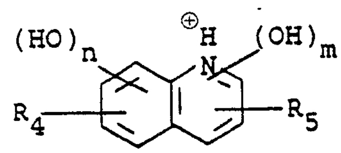

or a cation having the formula

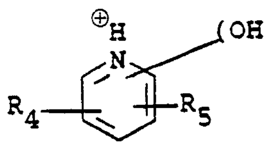,

with the proviso that if the cation used does not contain a hydroxy substituent, a nucleophile having the formula

$$R_6\text{—OH}$$

must also be present, the reaction being effected in the presence of an organic solvent (which may be the nucleophile $R_6$—OH), wherein

$R_1$ is an acyl group derived from a carboxylic acid;

$R_2$ and $R_3$ are the same or different and each is hydrogen, alkyl, alkenyl, alkynyl, cycloalkyl, phenyl, substituted phenyl or a 4, 5, 6 or 7-membered heterocycle, or one of $R_2$ and $R_3$ is hydrogen and the other is azido, halomethyl, dihalomethyl, trihalomethyl, alkoxycarbonyl, 2-phenylethenyl, 2-phenylethynyl, —$CH_2X_1$, carboxyl, —S—$X_2$, —O—$X_2$

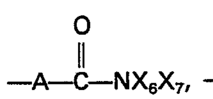, 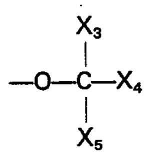 or 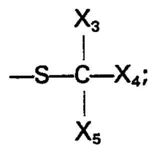;

wherein $X_1$ is azido, amino, hydroxy, alkanoylamino, phenylcarbonylamino, (substituted phenyl)carbonylamino, alkylsulfonyloxy, phenylsulfonyloxy, (substituted phenyl)sulfonyloxy, phenyl, substituted phenyl, cyano,

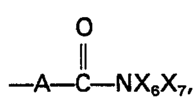,

—S—$X_2$ or —O—$X_2$; $X_2$ is alkyl, substituted alkyl, phenyl, substituted phenyl, phenylalkyl, (substituted phenyl)alkyl, alkanoyl, phenylalkanoyl, (substituted phenyl)alkanoyl, phenylcarbonyl, (substituted phenyl)carbonyl, or heteroarylcarbonyl) one of $X_3$ and $X_4$ is hydrogen and the other is hydrogen or alkyl, or

$X_3$ and $X_4$ when taken together with the carbon atom to which they are attached form a cycloalkyl group; $X_5$ is formyl, alkanoyl, phenylcarbonyl, (substituted phenyl)carbonyl, phenylalkylcarbonyl, (substituted phenyl)alkylcarbonyl, carboxyl, alkoxycarbonyl, aminocarbonyl, (substituted amino)carbonyl, or cyano; A is $-CH=CH-$, $-(CH_2)_n-$, $-CH_2-O-$, $-CH_2-NH-$ or $-CH_2-S-CH_2$; n is 0, 1 or 2; and $X_6$ and $X_7$ are the same or different and each is hydrogen, alkyl, phenyl or substituted phenyl, or $X_6$ is hydrogen and $X_7$ is amino, substituted amino, alkanoylamino or alkoxy, or $X_6$ and $X_7$ when taken together with the nitrogen atom to which they are attached form a 4, 5, 6 or 7-membered heterocycle;

$R_4$ and $R_5$ are the same or different and each is hydrogen, alkyl, substituted alkyl, phenyl, substituted phenyl, halogen, alkoxy, phenyloxy, (phenylcarbonyl)oxy, alkanoyloxy, alkanoylamino or (phenylcarbonyl)amino;

$R_6$ is hydrogen, alkyl, or phenyl;

n is 0 or 1, m is 0 or 1 and the sum of n + m is 0 or 1; and

p is 0 or 1,

wherein the terms "alkyl" and "alkoxy" refer to groups having 1 to 10 carbon atoms;

the term "cycloalkyl" refers to groups having 3, 4, 5, 6 or 7 carbon atoms;

the term "substituted alkyl" refers to alkyl groups substituted with one or more azido, amino, halogen, hydroxy, carboxyl, cyano, alkoxycarbonyl, aminocarbonyl, alkanoyloxy, alkoxy, phenyloxy (substituted phenyl)oxy, (a 4, 5, 6 or 7-membered heterocycle)oxy, mercapto, alkylthio, phenylthio, (substituted phenyl)thio, alkylsulfinyl or alkylsulfonyl groups;

the terms "alkanoyl", "alkenyl" and "alkynyl" refer to groups having 2 to 10 carbon atoms;

the term "substituted phenyl" refers to a phenyl group substituted with 1, 2, or 3 amino halogen, hydroxyl, trifluoromethyl, alkyl of 1 to 4 carbon atoms, alkoxy of 1 to 4 carbon atoms or carboxyl groups;

the term "substituted amino" refers to a group having the formula $-NY_1Y_2$ wherein $Y_1$ is hydrogen, alkyl, phenyl, substituted phenyl, phenylalkyl or (substituted phenyl)alkyl and $Y_2$ is alkyl, phenyl, substituted phenyl, phenylalkyl, (substituted phenyl)alkyl, hydroxy, cyano, alkoxy, phenylalkoxy or amino; and

the term "a 4, 5, 6 or 7-membered heterocycle" refers to pyridinyl, furanyl, pyrrolyl, thienyl, 1,2,3-triazolyl, 1,2,4-triazolyl, imidazolyl, thiazolyl, thiadiazolyl, pyrimidinyl, oxazoyl, triazinyl, tetrazolyl, azetinyl, oxetanyl, thietanyl, piperidinyl, piperazinyl, imidazolidinyl, oxazolidinyl, pyrrolidinyl, tetrahydropyrimidinyl, dihydrothiazolyl or hexahydroazepinyl or one of the above groups substituted with one or more oxo, halogen, hydroxy, nitro, amino, cyano, trifluoromethyl, alkyl of 1 to 4 carbon atoms, alkoxy of 1 to 4 carbon atoms, alkylsulfonyl, phenyl, substituted phenyl, 2-furylidineamino, benzylideneamino or substituted alkyl, wherein the alkyl group has 1 to 4 carbon atoms, groups.

2. A process in accordance with claim 1 wherein the anion and cation are in the form of a salt having the formula

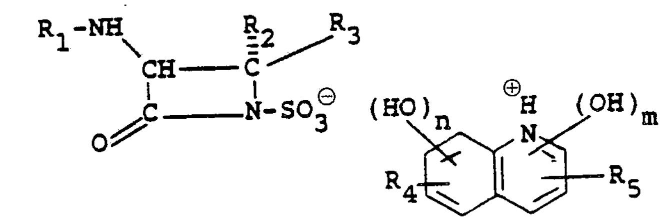

or

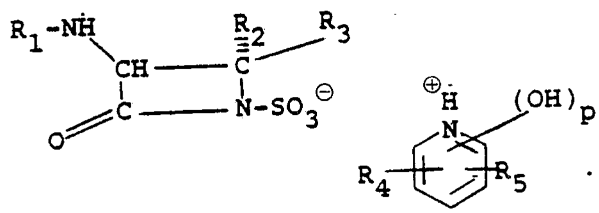

3. A process in accordance with claim 1 wherein the anion and cation are in the form of a salt having the formula

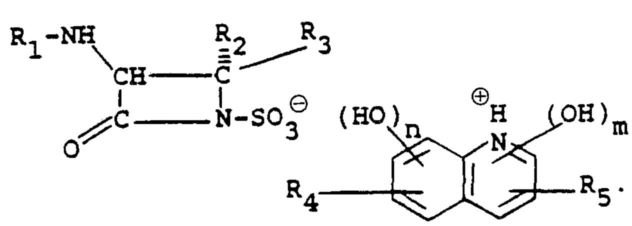

4. A process in accordance with claim 1 wherein the anion and cation are in the form of a salt having the formula

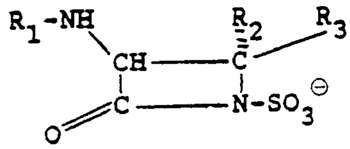 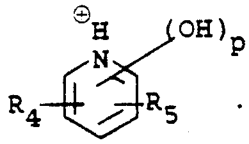 .

5. A process in accordance with claim 3 wherein the anion and cation are in the form of a salt having the formula

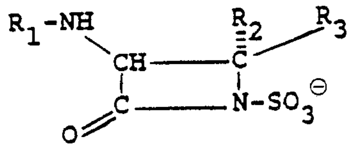 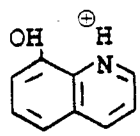 .

6. A process in accordance with claim 3 wherein the anion and cation are in the form of a salt having the formula

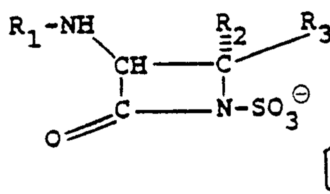 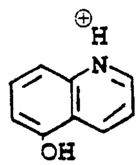 .

7. A process in accordance with claim 4 wherein the anion and cation are in the form of a salt having the formula

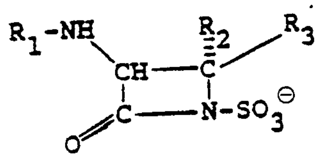 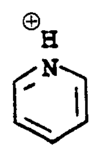

8. A process in accordance with claim 1 wherein one of $R_2$ and $R_3$ is hydrogen and the other is methyl.

9. A process in accordance with claim 2 wherein one of $R_2$ and $R_3$ is hydrogen and the other is methyl.

10. A process in accordance with claim 1 wherein $R_2$ and $R_3$ are each hydrogen.

11. A process in accordance with claim 2 wherein $R_2$ and $R_3$ are each hydrogen.

12. A process in accordance with claim 1 wherein the anion is in the form of a basic salt of a compound having the formula

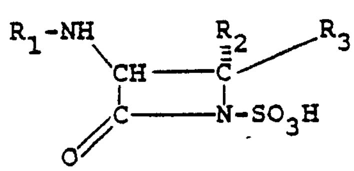

and the cation is in the form of an acid addition salt of a compound having the formula

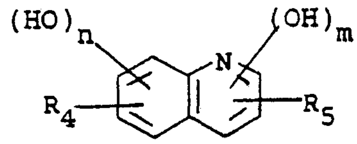 or 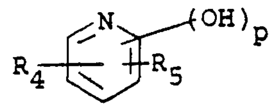 .

13. A process in accordance with claim 12 wherein the cation is in the form of a acid salt of a compound having the formula

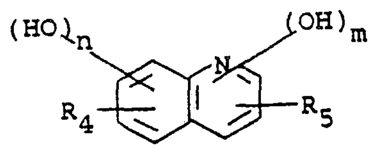 .

14. A process in accordance with claim 12 wherein the cation is in the form of an acid salt of 8-hydroxyquinoline.

15. A process in accordance with claim 12 wherein the cation is in the form of an acid addition salt of a compound having the formula

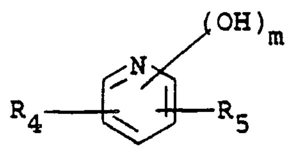 .

16. A process in accordance with claim 15 wherein the cation is in the form of an acid addition salt of pyridine.

17. A process in accordance with claim 15 wherein the cation is in the form of an acid addition salt of pyridine and the nucleophile used is ethanol.

**Patentansprüche**

1. Verfahren zur Herstellung einer Verbindung der Formel

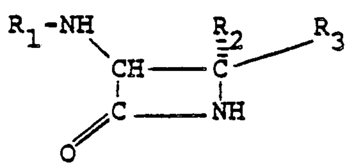

umfassend die Erwärmung eines Anions der Formel

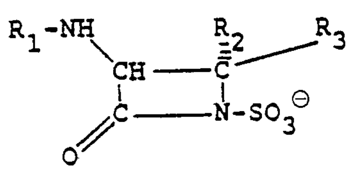

mit einem Kation der Formel

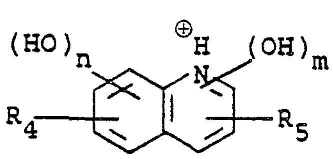

oder einem Kation der Formel

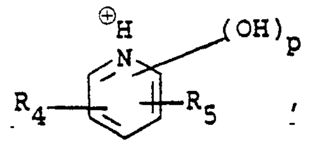 IV

mit der Massgabe, dass, wenn das verwendete Kation keinen Hydroxysubstituenten enthält, auch ein nucleophiles Agens der Formel

$$R_6—OH$$

vorhanden sein muss, wobei die Umsetzung in Gegenwart eines organischen Lösungsmittels (bei dem es sich um das nucleophile Agens $R_6$—OH handeln kann) durchgeführt wird, wobei $R_1$ einen von einer Carbonsäure abgeleiteten Acylrest bedeutet;

$R_2$ und $R_3$ gleich oder verschieden sind und jeweils Wasserstoff, Alkyl, Alkenyl, Alkinyl, Cycloalkyl, Phenyl, substituiertes Phenyl oder einen 4-, 5-, 6- oder 7-gliedrigen Heterocyclus bedeutet oder einer der Reste $R_2$ und $R_3$ Wasserstoff und der andere Azido, Halogenmethyl, Dihalogenmethyl, Trihalogenmethyl, Alkoxycarbonyl, 2-Phenyläthenyl, 2-Phenyläthinyl, —$CH_2X_1$, Carboxyl, —S—$X_2$, —O—$X_2$

$$—A—C(=O)—NX_6X_7,\quad —O—C(X_3)(X_5)—X_4 \quad \text{oder} \quad —S—C(X_3)(X_5)—X_4;$$

bedeutet; wobei $X_1$ Azido, Amino, Hydroxy, Alkanoylamino, Phenylcarbonylamino, (subst.-Phenyl)-carbonylamino, Alkylsulfonyloxy, Phenylsulfonyloxy, (subst.-Phenyl)-sulfonyloxy, Phenyl, substituiertes Phenyl, Cyano,

$$—A—C(=O)—NX_6X_7,$$

—S—$X_2$ oder —O—$X_2$ bedeutet; $X_2$ Alkyl, substituiertes Alkyl, Phenyl, substituiertes Phenyl, Phenylalkyl, (subst.-Phenyl)-alkyl, Alkanoyl, Phenylalkanoyl, (subst.-Phenyl)-alkanoyl, Phenylcarbonyl, (subst. Phenyl)-carbonyl oder Heteroarylcarbonyl bedeutet; einer der Reste $X_3$ und $X_4$ Wasserstoff und der andere Wasserstoff oder Alkyl bedeutet oder $X_3$ und $X_4$ zusammen mit dem Kohlenstoffatom, an das sie gebunden sind, einen Cycloalkylrest bilden; $X_5$ Formyl, Alkanoyl, Phenylcarbonyl, (subst.-Phenyl)-carbonyl, Phenylalkylcarbonyl, (subst.-Phenyl)-alkylcarbonyl, Carboxyl, Alkoxycarbonyl, Aminocarbonyl, (subst.-Amino)-carbonyl oder Cyano bedeutet; A —CH=CH—, —$(CH_2)_n$—, —$CH_2$—O—, —$CH_2$—NH— oder —$CH_2$—S—$CH_2$ bedeutet; n 0, 1 oder 2 ist; und $X_6$ und $X_7$ gleich oder verschieden sind und jeweils Wasserstoff, Alkyl, Phenyl oder substituiertes Phenyl bedeuten oder $X_6$ Wasserstoff und $X_7$ Amino, substituiertes Amino, Alkanoylamino oder Alkoxy bedeuten oder $X_6$ und $X_7$ zusammen mit dem Stickstoffatom, an das sie gebunden sind, einen 4-, 5-, 6- oder 7-gliedrigen Heterocyclus bilden;

$R_4$ und $R_5$ gleich oder verschieden sind und jeweils Wasserstoff, Alkyl, substituiertes Alkyl, Phenyl, substituiertes Phenyl, Halogen, Alkoxy, Phenyloxy, (Phenylcarbonyl)-oxy, Alkanoyloxy, Alkanoylamino oder (Phenylcarbonyl)-amino bedeuten;

$R_6$ Wasserstoff, Alkyl oder Phenyl bedeutet;

n 0 oder 1 ist, m 0 oder 1 ist und die Summe von n + m 0 oder 1 ist; und

p 0 oder 1 ist,

wobei die Ausdrücke "Alkyl" und "Alkoxy" sich auf Reste mit 1 bis 10 Kohlenstoffatomen beziehen;

der Ausdruck "Cycloalkyl" sich auf Reste mit 3, 4, 5, 6 oder 7 Kohlenstoffatomen bezieht;

der Ausdruck "substituiertes Alkyl" sich auf Alkylreste bezieht, die ein- oder mehrfach durch Azido, Amino, Halogen, Hydroxy, Carboxyl, Cyano, Alkoxycarbonyl, Aminocarbonyl, Alkanoyloxy, Alkoxy, Phenyloxy, (subst.-Phenyl)-oxy, 4-, 5-, 6- oder 7-gliedriges (Heterocyclyl)-oxy, Mercapto, Alkylthio, Phenylthio, (subst.-Phenyl)-thio, Alkylsulfinyl oder Alkylsulfonyl substituiert sind;

die Ausdrücke "Alkanoyl", "Alkenyl" und "Alkinyl" sich auf Reste mit 2 bis 10 Kohlenstoffatomen beziehen;

der Ausdruck "substituiertes Phenyl" sich auf einen Phenylrest bezieht, der 1-, 2-, oder 3-fach durch Amino, Halogen, Hydroxyl, Trifluormethyl, Alkyl mit 1 bis 4 Kohlenstoffatomen, Alkoxy mit 1 bis 4 Kohlenstoffatomen oder Carboxyl substituiert ist;

der Ausdruck "substituiertes Amino" sich auf einen Rest der Formel —$NY_1Y_2$ bezieht, worin $Y_1$ Wasserstoff, Alkyl, Phenyl, substituiertes Phenyl, Phenylalkyl oder (subst.-Phenyl)-alkyl und $Y_2$ Alkyl, Phenyl, substituiertes Phenyl, Phenylalkyl, (subst.-Phenyl)-alkyl, Hydroxy, Cyano, Alkoxy, Phenylalkoxy oder Amino bedeuten; und

der Ausdruck "4-, 5-, 6- oder 7-gliedriger Heterocyclus" sich auf Pyridinyl, Furanyl, Pyrrolyl, Thienyl, 1,2,3-Triazolyl, 1,2,4-Triazolyl, Imidazolyl, Thiazolyl, Thiadiazolyl, Pyrimidinyl, Oxazoyl, Triazinyl, Tetrazolyl, Azetinyl, Oxetanyl, Thietanyl, Piperidinyl, Piperazinyl, Imidazolidinyl, Oxazolidinyl, Pyrrolidinyl, Tetrahydropyrimidinyl, Dihydrothiazolyl oder Hexahydroazepinyl oder auf einen der vorstehenden Reste, die ein- oder mehrfach durch Oxo, Halogen, Hydroxy, Nitro, Amino, Cyano, Trifluormethyl, Alkyl mit 1 bis 4 Kohlenstoffatomen, Alkoxy mit 1 bis 4 Kohlenstoffatomen, Alkylsulfonyl, Phenyl, substituiertes Phenyl, 2-Furylidinamino, Benzylidenamino oder substituiertes Alkyl, wobei der Alkylrest 1 bis 4 Kohlenstoffatome aufweist, substituiert sind, bezieht.

2. Verfahren nach Anspruch 1, wobei das Anion und das Kation in Form eines Salzes der Formel

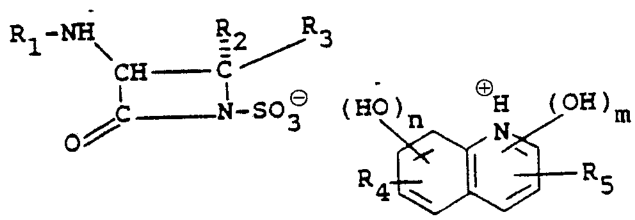

oder

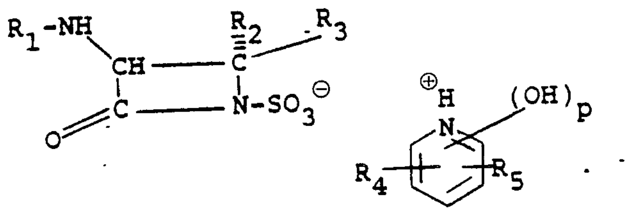

vorliegen.

3. Verfahren nach Anspruch 1, wobei das Anion und das Kation in Form eines Salzes der Formel

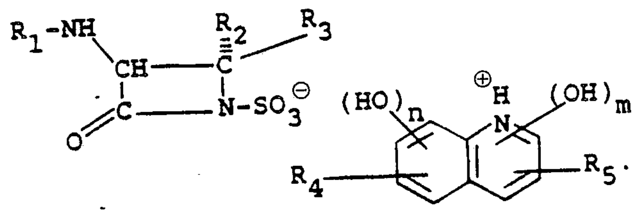

vorliegt.

4. Verfahren nach Anspruch 1, wobei das Anion und das Kation in Form eines Salzes der Formel

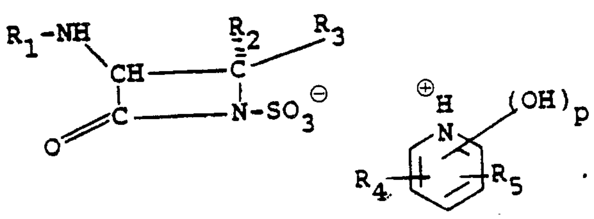

vorliegt.

5. Verfahren nach Anspruch 3, wobei das Anion und das Kation in Form eines Salzes der Formel

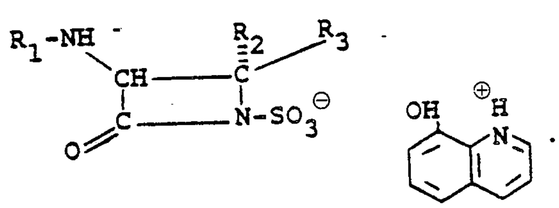

vorliegt.

6. Verfahren nach Anspruch 3, wobei das Anion und das Kation in Form eines Salzes der Formel

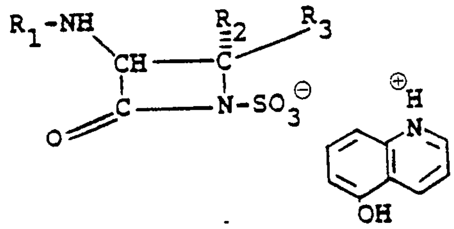
.

vorliegt.

7. Verfahren nach Anspruch 4, wobei das Anion und das Kation in Form eines Salzes der Formel

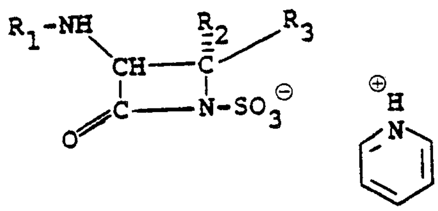

vorliegt.

8. Verfahren nach Anspruch 1, wobei einer der Reste $R_2$ und $R_3$ Wasserstoff und der andere Methyl bedeutet.

9. Verfahren nach Anspruch 2, wobei einer der Reste $R_2$ und $R_3$ Wasserstoff und der andere Methyl bedeutet.

10. Verfahren nach Anspruch 2, wobei $R_2$ und $R_3$ jeweils Wasserstoff bedeuten.

11. Verfahren nach Anspruch 2, wobei $R_2$ und $R_3$ jeweils Wasserstoff bedeuten.

12. Verfahren nach Anspruch 1, wobei das Anion in Form eines basischen Salzes einer Verbindung der Formel

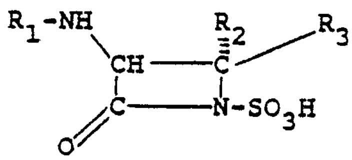

vorliegt und das Kation in Form eines Säureadditionssalzes einer Verbindung der Formel

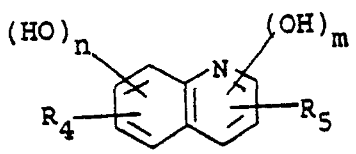
oder
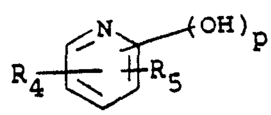
.

vorliegt.

13. Verfahren nach Anspruch 12, wobei das Kation in Form eines Säureadditinossalzes einer Verbindung der Formel

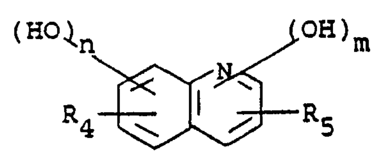
.

vorliegt.

14. Verfahren nach Anspruch 12, wobei das Kation in Form eines Säureadditionssalzes von 8-Hydroxychinolin vorliegt.

15. Verfahren nach Anspruch 12, wobei das Kation in Form eines Säureadditionssalzes einer Verbindung der Formel

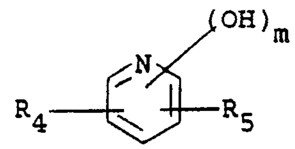

vorliegt.

16. Verfahren nach Anspruch 15, wobei das Kation in Form eines Säureadditionssalzes von Pyridin vorliegt.

17. Verfahren nach Anspruch 15, wobei das Kation in Form eines Säureadditionssalzes von Pyridin vorliegt und als nucleophiles Agens Äthanol verwendet wird.

**Revendications**

1. Procédé de préparation d'un composé de formule

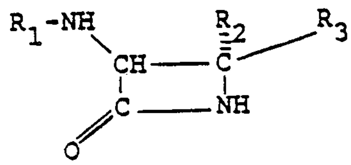

qui consiste à chauffer un anion de formule

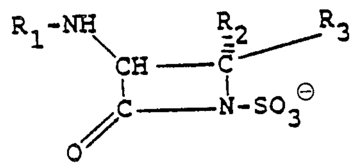

avec un cation de formule

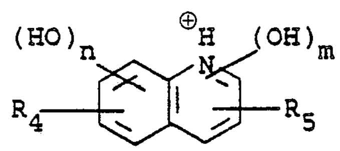

ou avec un cation de formule

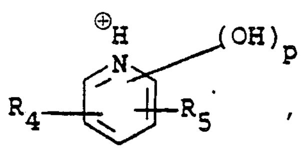

à condition que, si le cation utilisé ne contient pas de substituant hydroxy, un nucléophile de formule

$$R_6—OH$$

soit également présent, la réaction étant effectuée en présence d'un solvant organique (qui peut être le nucléophile $R_6$—OH), où

$R_1$ est un radical acyle dérivé d'un acide carboxylique;

$R_2$ et $R_3$ sont identiques ou différents et sont chacun un atome d'hydrogène, un radical alkyle, alcényle, alcynyle, cycloalkyle, phényle ou phényle substitué, ou un hétérocycle à 4, 5, 6 ou 7 chaînons, ou bien l'un de $R_2$ et $R_3$ est un atome d'hydrogène et l'autre est un radical azide, halométhyle, dihalométhyle, trihalométhyle, alcoxycarbonyle, 2-phényléthényle, 2-phényléthynyle —$CH_2X_1$, carboxyle, —S—$X_2$, —O—$X_2$,

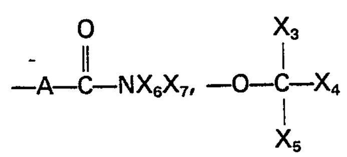 ou 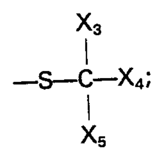;

où $X_1$ est un radical azide, amine, hydroxy, alcanoylamine, phénylcarbonylamine, (phényl substitué)-carbonylamine, alkylsulfonyloxy, phénylsulfonyloxy, (phényl substitué)sulfonyloxy, phényle, phényle substitué, cyano,

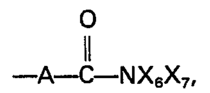

$—S—X_2$ ou $—O—X_2$; $X_2$ est un radical alkyle, alkyle substitué, phényle, phényle substitué, phénylalkyle, (phényl substitué)alkyle, alcanoyle, phénylalcanoyle, (phényl substitué)alcanoyle, phénylcarbonyle, (phényl substitué)carbonyle, ou hétéroarylcarbonyle; l'un de $X_3$ et $X_4$ est un atome d'hydrogène et l'autre est un atome d'hydrogène ou un radical alkyle, ou bien $X_3$ et $X_4$ forment, avec l'atome de carbone auquel ils sont fixés, un radical cycloalkyle; $X_5$ est un radical formyle, alcanoyle, phénylcarbonyle, (phényl substitué)-carbonyle, phénylalkylcarbonyle, (phénylsubstitué)alkylcarbonyle, carboxyle, alcoxycarbonyle, aminocarbonyle, (amino substitué)carbonyle, ou cyano; A est $—CH{=}CH$, $—(CH_2)_n—$, $—CH_2—O—$, $—CH_2—NH—$ ou $—CH_2—S—CH_2$; n est égal à 0, 1 ou 2; et $X_6$ et $X_7$ sont identiques ou différents et sont chacun un atome d'hydrogène ou un radical alkyle, phényle ou phényle substitué, ou bien $X_6$ est un atome d'hydrogène et $X_7$ est un groupement amine, amine substitué, alcanoylamine ou alcoxy, ou encore $X_6$ et $X_7$ forment, avec l'atome d'azote auquel ils sont fixés, un hétérocycle à 4, 5, 6 ou 7 chaînons;

$R_4$ et $R_5$ sont identiques ou différents et sont chacun un atome d'hydrogène, un radical alkyle, alkyle substitué, phényle, phényle substitué, un atome d'halogène, ou un radical alcoxy, phényloxy, (phénylcarbonyl)oxy, alcanoyloxy, alcanoylamine ou (phénylcarbonyl)amine;

$R_6$ est un atome d'hydrogène ou un radical alkyle ou phényle;

n est égal à 0 ou 1, m est égal à 0 ou 1, et la somme n + m est égale à 0 ou 1; et

p est égal à 0 ou 1,

où les termes "alkyle" et "alcoxy" désignent des groupements ayant de 1 à 10 atomes de carbone;

le terme "cycloalkyle" désigne des groupements ayant 3, 4, 5, 6 ou 7 atomes de carbone;

le terme "alkyle substitué" désigne des radicaux alkyle substitués par un ou plusieurs atomes, radicaux ou groupements azide, amine, halogène, hydroxy, carboxyle, cyano, alcoxycarbonyle, aminocarbonyle, alcanoyloxy, alcoxy, phényloxy, (phényl substitué)oxy, (hétérocycle à 4, 5, 6 ou 7 chaînons)oxy, mercapto, alkylthio, phénylthio, (phényl substitué)thio, alkylsulfinyle ou alkylsulfonyle;

les termes "alcanoyle", "alcényle" et "alcynyle" désignent des radicaux ayant de 2 à 10 atomes de carbone;

le terme "phényle substitué" désigne un radical phényle substitué par 1, 2 ou 3 atomes, radicaux ou groupements amine, halogène, hydroxyle, trifluorométhyle, alkyle de 1 à 4 atomes de carbone, alcoxy de 1 à 4 atomes de carbone, ou carboxyle;

le terme "amine substitué" désigne un groupement de formule $—NY_1Y_2$ dans laquelle $Y_1$ est un atome d'hydrogène ou un radical alkyle, phényle, phényle substitué, phénylalkyle ou (phényl substitué)alkyle et $Y_2$ est un radical alkyle, phényle, phényle substitué, phénylalkyle, (phényl substitué)alkyle, hydroxy, cyano, alcoxy, phénylalcoxy ou amine; et

le terme "hétérocycle à 4, 5, 6 ou 7 chaînons" désigne un radical pyridinyle, furanyle, pyrrolyle, thiényle, 1,2,3-triazolyle, 1,2,4-triazolyle, imidazolyle, thiazolyle, thiadiazolyle, pyrimidinyle, oxazolyle, triazinyle, tétrazolyle, azétinyle, oxétanyle, thiétanyle, pipéridinyle, pipérazinyle, imidazolidinyle, oxazolidinyle, pyrrolidinyle, tétrahydropyrimidinyle, dihydrothiazolyle ou hexahydroazépinyle, ou bien l'un des radicaux précédents substitué par un ou plusieurs atomes, radicaux ou groupements oxo, halogène, hydroxy, nitro, amine, cyano, trifluorométhyle, alkyle de 1 à 4 atomes de carbone, alcoxy de 1 à 4 atomes de carbone, alkylsulfonyle, phényle, phényle substitué, 2-furylidèneamine, benzylidèneamine ou alkyle substitué, où le radical alkyle a de 1 à 4 atomes de carbone.

2. Procédé selon la revendication 1, dans lequel l'anion et le cation se présentent sous la forme d'un sel de formule

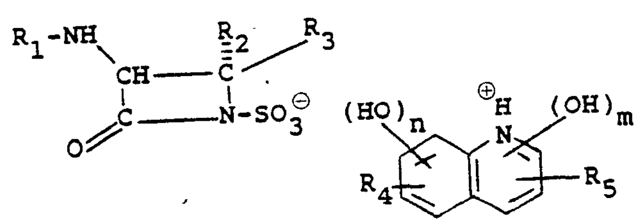

ou

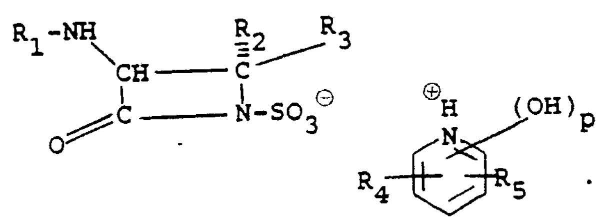

3. Procédé selon la revendication 1, dans lequel l'anion et le cation se présentent sous la forme d'un sel de formule

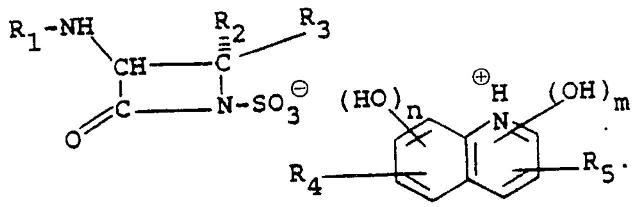

4. Procédé selon la revendication 1, dans lequel l'anion et le cation se présentent sous la forme d'un sel de formule

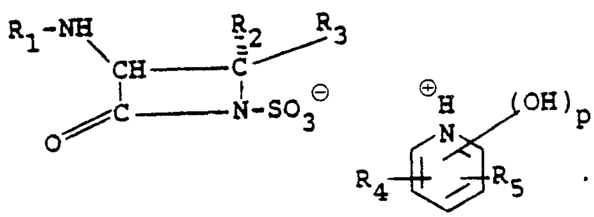

5. Procédé selon la revendication 3, dans lequel l'anion et le cation se présentent sous la forme d'un sel de formule

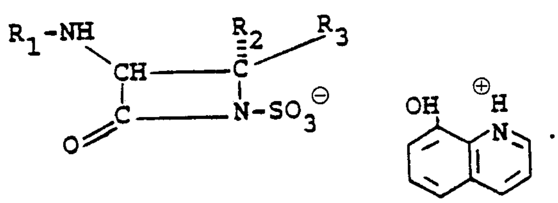

6. Procédé selon la revendication 3, dans lequel l'anion et le cation se présentent sous la forme d'un sel de formule

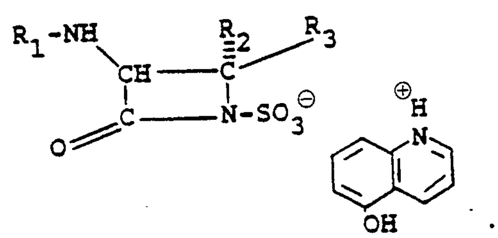

7. Procédé selon la revendication 4, dans lequel l'anion et le cation se présentent sous la forme d'un sel de formule

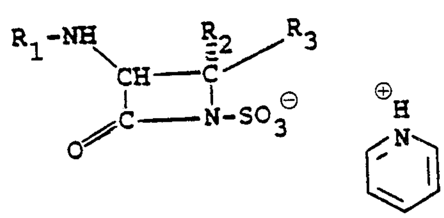

8. Procédé selon la revendication 1, dans lequel l'un de $R_2$ et $R_3$ est un atome d'hydrogène et l'autre est le radical méthyle.

9. Procédé selon la revendication 2, dans lequel l'un de $R_2$ et $R_3$ est un atome d'hydrogène et l'autre est le radical méthyle.

10. Procédé selon la revendication 1, dans lequel $R_2$ et $R_3$ sont tous deux un atome d'hydrogène.

11. Procédé selon la revendication 2, dans lequel $R_2$ et $R_3$ sont tous deux un atome d'hydrogène.

12. Procédé selon la revendication 1, dans lequel l'anion se présente sous la forme d'un sel basique d'un composé de formule

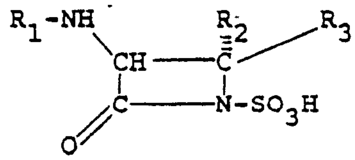

et le cation se présente sous la forme d'un sel d'addition d'acide d'un composé de formule

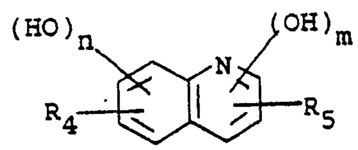

ou

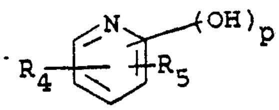

13. Procédé selon la revendication 12, dans lequel le cation se présente sous la forme d'un sel d'addition d'acide d'un composé de formule

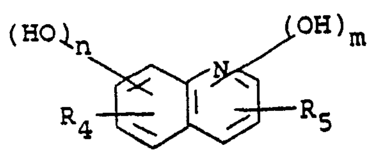

14. Procédé selon la revendication 12, dans lequel le cation se présente sous la forme d'un sel d'addition d'acide de la 8-hydroxyquinoléine.

15. Procédé selon la revendication 12, dans lequel le cation se présente sous la forme d'un sel d'addition d'acide d'un composé de formule

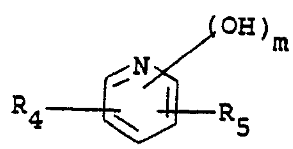

16. Procédé selon la revendication 15, dans lequel le cation se présente sous la forme d'un sel d'addition d'acide de la pyridine.

17. Procédé selon la revendication 15, dans lequel le cation se présente sous la forme d'un sel d'addition d'acide de la pyridine, et le nucléophile utilisé est l'éthanol.